# EUROPEAN PATENT APPLICATION

(11) **EP 4 814 133 A1**
(43) Date of publication of application: **30.09.2026**
(21) Application number: 24893634.6
(22) Date of filing: 25.11.2024
(51) Int. Cl.: C12Q 1/689, C12Q 1/6844, C12N 15/11

(54) **METHOD FOR RAPIDLY DETECTING MYCOBACTERIUM TUBERCULOSIS**

(30) Priority: 24.11.2023 CN 202311588804
(71) Applicant: ShanghaiTech University, Shanghai 201210 (CN); Ruijin Hospital, Shanghai Jiao Tong University School of Medecine, Shanghai 200025 (CN)
(72) Inventor: LIU, Jia, Shanghai 201210 (CN); QU, Jieming, Shanghai 200025 (CN)
(74) Representative: Maiwald GmbH
(86) International application number: PCT/CN2024/134194
(87) International publication number: WO 2025/108481

(57) **Abstract**

Provided is a method for rapidly detecting Mycobacterium tuberculosis. The method comprises using a primer pair for detecting Mycobacterium tuberculosis, which primer pair comprising an upstream primer and a downstream primer; wherein the upstream primer comprises a nucleotide sequence as shown in SEQ ID NO: 1 or 2, and the downstream primer comprises a nucleotide sequence as shown in any one of SEQ ID NOs: 3-6. The method can rapidly and accurately detect the RNA of MTB viable bacteria in a biological sample with high sensitivity, reduces the detection limit by up to 1000 times compared with existing NASBA kits, reduces the requirements for templates, and provides a better option for the real-time detection of MTB.

## Description

The present application claims the priority of Chinese patent application 2023115888043 filed on November 24, 2023. The contents of the Chinese patent application are incorporated herein by reference in their entireties.

### TECHNICAL FIELD

The present disclosure belongs to the field of biological detection, and specifically relates to a method for rapid detection of mycobacteria.

### BACKGROUND

Tuberculosis (TB), caused by *Mycobacterium tuberculosis* (MTB), is a major public health concern. Clinical practice has demonstrated that effective diagnosis of TB plays a critical role in the prevention and control of the disease. Traditional diagnostic methods for TB include etiological, immunological and nucleic acid detection approaches. Microbial culture is generally recognized as the gold standard for etiological diagnosis of TB. Although it enables convenient detection of viable *Mycobacterium tuberculosis,* culture methods may require up to 8 weeks and multiple clinical visits, leading to delays in TB treatment. The tuberculin skin test and interferon-gamma release assay are important auxiliary diagnostic methods for TB, which rely on MTB antigens or the response of lymphocytes to antigens, respectively. However, the results of these immunological tests can be complicated by the patient's immune status, for example, after Bacillus Calmette-Guérin (BCG) vaccination or co-infection with human immunodeficiency virus (HIV).

Recently, the World Health Organization (WHO) has emphasized nucleic acid amplification tests (NAATs) for TB diagnosis, including the PCR-based Xpert MTB/RIF technology. A drawback of Xpert MTB/RIF testing is that the economic cost of the accompanying components (the GeneXpert system) hinders its widespread use in low-income countries or regions, which actually bear a high risk of MTB infection. Isothermal amplification-based NAATs, such as loop-mediated isothermal amplification (LAMP) and nucleic acid sequence-based amplification (NASBA), have been developed to alleviate the demand for infrastructure. However, these NAATs have limited ability to distinguish between dead and viable MTB.

To overcome these shortcomings, rapid and low-cost methods for the specific detection of viable MTB have been developed. These novel diagnostic methods include modified propidium monoazide (PMA) treatment that allows selective PCR amplification of DNA from viable MTB, fluorescent chemical probes that can be activated in response to MTB proteins, induced expression of specific MTB proteins, and bioaerosol capture technology.

Recent studies have shown that the clustered regularly interspaced short palindromic repeat (CRISPR)-associated (Cas) proteins (CRISPR-Cas) can be used as a rapid nucleic acid detection technology. CRISPR-based diagnosis relies on the collateral cleavage activity of Cas nucleases, which can induce the cleavage of single-stranded oligonucleotide reporters to generate fluorescent or immunogenic signals. Several studies have described the detection of MTB DNA based on CRISPR-Cas12. These diagnostic methods can be implemented via fluorescent signals and lateral flow, thus enabling point-of-care testing (POCT) of MTB. Notably, CRISPR-based diagnosis can be used to detect cell-free circulating MTB DNA in human serum even in the case of HIV co-infection. In addition, CRISPR-Cas13a has been applied to identify drug resistance mutations in MTB with single-nucleotide precision. Despite significant advances in CRISPR-based MTB diagnosis, distinguishing between dead and viable MTB remains challenging.

### SUMMARY

The technical problem to be solved by the present disclosure is to address the defects of the prior art, such as high false negative rate, low sensitivity for pathogenic *Mycobacterium* species, and long time consumption in MTB detection, and to provide a method for rapid detection of mycobacteria. The present disclosure enables rapid, accurate and high-sensitivity detection of MTB RNA in biological samples, distinguishes MTB from non-tuberculous mycobacteria (NTM), specific and non-specific Mycobacterium species, and provides a better option for point-of-care testing of MTB.

The present disclosure describes a CRISPR-Cas13a-based molecular diagnostic method that serves as a diagnostic platform for the specific detection of MTB single-stranded RNA (ssRNA) rather than genomic double-stranded DNA (dsDNA), enabling rapid and specific detection of viable MTB. This method, termed CRISPR-Live-MTB in the present disclosure, comprises two consecutive reactions: nucleic acid sequence-based amplification (NASBA) and CRISPR-Cas13a collateral cleavage reaction. Experiments show that CRISPR-Live-MTB can effectively detect MTB ssRNA within 2 hours, with higher specificity than dsDNA detection. Importantly, CRISPR-Live-MTB exhibits a limit of detection (LOD) as low as 2.4 copies for MTB ssRNA, which is 1000-fold lower than that of the NASBA-only method (2,400 copies), greatly reducing the risk of missed detection. At this LOD, the template concentration required for CRISPR-Live-MTB is only 0.2 aM, whereas that for standalone NASBA is 0.2 fM. CRISPR-Live-MTB drastically improves the accuracy of MTB detection and reduces the risk of false negatives. In addition, CRISPR-Live-MTB incorporates lateral flow readout, enabling point-of-care testing (POCT) with 95% sensitivity and 100% specificity. Meanwhile, the inventors also tested the signal-to-noise ratio of RNA relative to DNA. These results demonstrate that the CRISPR-Live-MTB of the present disclosure can be used as a rapid, sensitive and specific method for the detection of viable MTB.

The present disclosure solves the aforementioned technical problems through the following technical solutions.

A first aspect of the present disclosure provides a primer pair for detecting *Mycobacterium tuberculosis;* the primer pair comprises an upstream primer and a downstream primer;

the upstream primer comprises a nucleotide sequence as shown in SEQ ID NO: 1 or 2, and the downstream primer comprises a nucleotide sequence as shown in any one of SEQ ID NOs: 3-6.

In some embodiments of the present disclosure, the upstream primer and the downstream primer are selected from the following combinations:
(1) the upstream primer comprises or is a nucleotide sequence as shown in SEQ ID NO: 1, and the downstream primer comprises or is a nucleotide sequence as shown in SEQ ID NO: 6;
(2) the upstream primer comprises or is a nucleotide sequence as shown in SEQ ID NO: 1, and the downstream primer comprises or is a nucleotide sequence as shown in SEQ ID NO: 4;
(3) the upstream primer comprises or is a nucleotide sequence as shown in SEQ ID NO: 2, and the downstream primer comprises or is a nucleotide sequence as shown in SEQ ID NO: 4;
(4) the upstream primer comprises or is a nucleotide sequence as shown in SEQ ID NO: 2, and the downstream primer comprises or is a nucleotide sequence as shown in SEQ ID NO: 6;
(5) the upstream primer comprises or is a nucleotide sequence as shown in SEQ ID NO: 1, and the downstream primer comprises or is a nucleotide sequence as shown in SEQ ID NO: 3;
(6) the upstream primer comprises or is a nucleotide sequence as shown in SEQ ID NO: 2, and the downstream primer comprises or is a nucleotide sequence as shown in SEQ ID NO:5;
(7) the upstream primer comprises or is a nucleotide sequence as shown in SEQ ID NO: 1, and the downstream primer comprises or is a nucleotide sequence as shown in SEQ ID NO: 5;
(8) the upstream primer comprises or is a nucleotide sequence as shown in SEQ ID NO: 2, and the downstream primer comprises or is a nucleotide sequence as shown in SEQ ID NO: 3.

In some embodiments of the present disclosure, the 5' end of the upstream primer further comprises a T7 promoter sequence; the T7 promoter sequence is shown, for example, in SEQ ID NO: 24.

A second aspect of the present disclosure provides a nucleic acid detection kit for nucleic acid-dependent amplification; the nucleic acid detection kit comprises the primer pair as described in the first aspect.

In some embodiments of the present disclosure, the nucleic acid detection kit further comprises a reverse transcriptase, an RNase inhibitor, and an RNA polymerase.

In some preferred embodiments of the present disclosure, the nucleic acid detection kit further comprises an RNA template.

In some preferred embodiments of the present disclosure, the RNA polymerase is T7 RNA polymerase.

A third aspect of the present disclosure provides a reaction system for nucleic acid-dependent amplification; the reaction system comprises the primer pair as described in the first aspect.

In some embodiments of the present disclosure, the working concentration of the primer pair is 0.05-1 µM, preferably 0.25-1 µM.

In some embodiments of the present disclosure, the reaction system further comprises a reverse transcriptase, an RNase inhibitor, and an RNA polymerase.

A fourth aspect of the present disclosure provides an amplification method for nucleic acid-dependent amplification; the amplification method comprises adding an RNA template to the reaction system as described in the third aspect to perform an amplification reaction.

In some embodiments of the present disclosure, the RNA template is a 16S rRNA template.

In some embodiments of the present disclosure, the working concentration of the RNA template is at least 0.2 aM.

In some embodiments of the present disclosure, the working concentration of the RNA template is 1 to 1000 times lower than the working concentration specified in the instructions of a NASBA kit such as NECB-24.

In some embodiments of the present disclosure, the temperature of the amplification reaction is 41-42°C.

A fifth aspect of the present disclosure provides a crRNA for detecting *Mycobacterium;* the crRNA comprises a spacer sequence of the nucleotide sequence as shown in any one of SEQ ID NOs: 9-21.

In some embodiments of the present disclosure, the spacer sequence is a nucleotide sequence of 28 bp in length located at the 3' end of the nucleotide sequence as shown in any one of SEQ ID NOs: 9-21.

In some specific embodiments of the present disclosure, the crRNA comprises a spacer sequence of the nucleotide sequence as shown in SEQ ID NO: 15.

A sixth aspect of the present disclosure provides a crRNA for detecting *Mycobacterium tuberculosis, Mycobacterium gordonae, Mycobacterium abscessus, Mycobacterium fortuitum, Mycobacterium avium, Mycobacterium intracellulare* or *Mycobacterium kansasii;* the crRNA comprises a spacer sequence of the nucleotide sequence as shown in any one of SEQ ID NOs: 9-14, SEQ ID NO: 16, SEQ ID NO: 17, SEQ ID NO: 18, SEQ ID NO: 19, SEQ ID NO: 20, or SEQ ID NO: 21, respectively.

In some embodiments of the present disclosure, the spacer sequence is a nucleotide sequence of 28 bp in length located at the 3' end of the nucleotide sequence.

A seventh aspect of the present disclosure provides a CRISPR detection kit; the CRISPR detection kit comprises the crRNA as described in the fifth or sixth aspect.

In some embodiments of the present disclosure, the CRISPR detection kit further comprises a Cas protein, such as LwaCas13a.

In some preferred embodiments of the present disclosure, the CRISPR detection kit further comprises an ssRNA fluorescent probe; the ssRNA fluorescent probe comprises, for example, a nucleotide sequence as shown in SEQ ID NO: 7.

An eighth aspect of the present disclosure provides a reaction system for CRISPR detection; the reaction system comprises the crRNA as described in the fifth or sixth aspect.

In some embodiments of the present disclosure, the working concentration of the crRNA is 0.1-2.5 ng/µL, preferably 0.5-2.5 ng/µL.

In some embodiments of the present disclosure, the reaction system further comprises a Cas protein, such as LwaCas13a.

In some embodiments of the present disclosure, the working concentration of the Cas protein is 45-50 nM.

In some embodiments of the present disclosure, the reaction system further comprises an ssRNA fluorescent probe; the ssRNA fluorescent probe comprises, for example, a nucleotide sequence as shown in SEQ ID NO: 7.

In some embodiments of the present disclosure, the working concentration of the ssRNA fluorescent probe is 0.25-0.5 µM.

A ninth aspect of the present disclosure provides a method for CRISPR detection; the method comprises a step of adding a sample to be tested to the reaction system as described in the eighth aspect to conduct a reaction.

In some embodiments of the present disclosure, the reaction temperature is 35-37°C.

In some embodiments of the present disclosure, the CRISPR detection is detection for non-diagnostic purposes, for example, laboratory testing of environmental samples or plant samples.

In some embodiments of the present disclosure, the CRISPR detection is an *in vitro* detection.

A tenth aspect of the present disclosure provides a method for detecting *Mycobacterium tuberculosis;* the method comprises steps of sequentially performing RNA nucleic acid-dependent amplification and Cas13a-based CRISPR detection on a sample to be tested; the RNA nucleic acid-dependent amplification comprises performing RNA amplification using the primer pair as described in the first aspect, the nucleic acid detection kit as described in the second aspect, or the reaction system as described in the third aspect.

In some embodiments of the present disclosure, the CRISPR detection comprises performing detection using the crRNA as described in the fifth or sixth aspect, the CRISPR detection kit as described in the seventh aspect or the reaction system as described in the eighth aspect.

In some embodiments of the present disclosure, the *Mycobacterium tuberculosis* is viable *Mycobacterium tuberculosis.*

In some embodiments of the present disclosure, the method is for non-diagnostic purposes.

In some preferred embodiments of the present disclosure, the reaction temperature for the RNA amplification is 41-42°C.

In some preferred embodiments of the present disclosure, a thermal activation step is further comprised before the RNA amplification; the thermal activation preferably comprises thermal activation of a nucleic acid and a nuclease inhibitor, and the temperature of the thermal activation is preferably 65-67°C.

In some embodiments of the present disclosure, the reaction temperature for the detection is 35-37°C.

An eleventh aspect of the present disclosure provides use of the primer pair as described in the first aspect, the nucleic acid detection kit as described in the second aspect, the reaction system as described in the third aspect, the crRNA as described in the fifth or sixth aspect, the CRISPR detection kit as described in the seventh aspect or the reaction system as described in the eighth aspect in the detection of pathogens of *Mycobacterium* or in the preparation of a reagent or device for detecting pathogens of *Mycobacterium.*

In some embodiments of the present disclosure, the pathogen of *Mycobacterium* is selected from *Mycobacterium tuberculosis, Mycobacterium gordonae, Mycobacterium abscessus, Mycobacterium fortuitum, Mycobacterium avium, Mycobacterium intracellulare* and *Mycobacterium kansasii.*

Any of the aforementioned preferred conditions can be combined arbitrarily on the basis of common general knowledge in the art to obtain preferred embodiments of the present disclosure.

All reagents and raw materials used in the present disclosure are commercially available.

The positive and progressive effects of the present disclosure are as follows:

Compared with existing NASBA detection, the CRISPR-Live-MTB of the present disclosure drastically reduces the limit of detection. For example, the LOD of the NASBA reaction is 2,400 copies (corresponding to a template concentration of 0.2 fM), while the LOD of CRISPR-Live-MTB (1 hour NASBA + 1 hour CRISPR reaction) is only 2.4 copies (corresponding to a template concentration of 0.2 aM). This indicates that CRISPR-Live-MTB can significantly reduce the template concentration, greatly improve the accuracy of MTB detection, and reduce the risk of false negatives.

Furthermore, CRISPR-Live-MTB can achieve point-of-care testing (POCT) with 95% sensitivity and 100% specificity within a short time, effectively monitor the efficacy of drug treatment and disease progression, and benefit patients in areas with inconvenient transportation and face-to-face consultations.

### BRIEF DESCRIPTION OF THE DRAWINGS

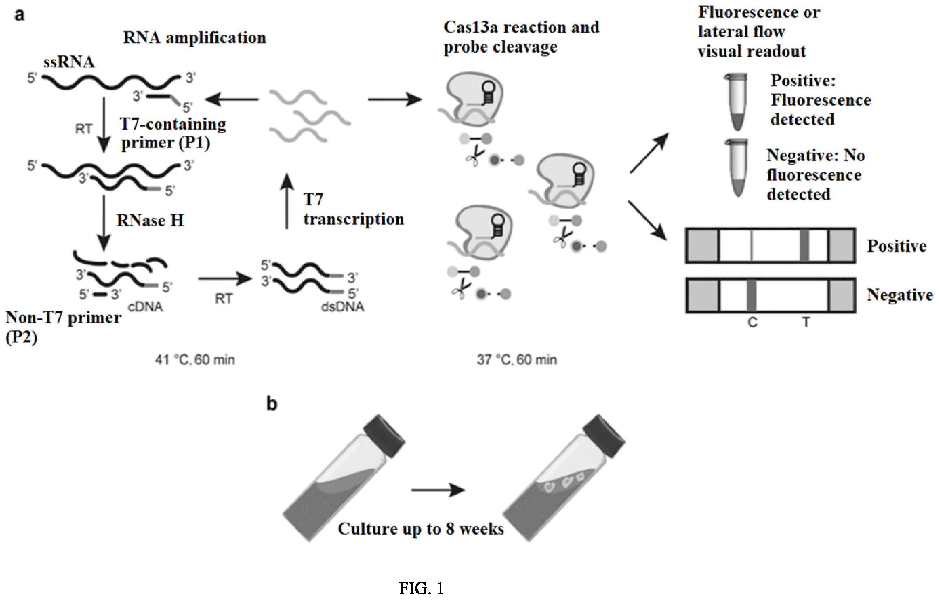

FIG. 1 is a schematic diagram of the CRISPR-Live-MTB of the present disclosure; in the figure: a shows the CRISPR-Live-MTB method, which designs CRISPR RNA (crRNA) targeting the 16S rRNA gene to detect MTB RNA. Conventional RNA extraction methods can be used as RNA input. Signal readings can be fluorescent signals or colorimetric bands on a lateral flow test strip; RT represents reverse transcription, and T7 represents the gray region; b shows the traditional culture method.

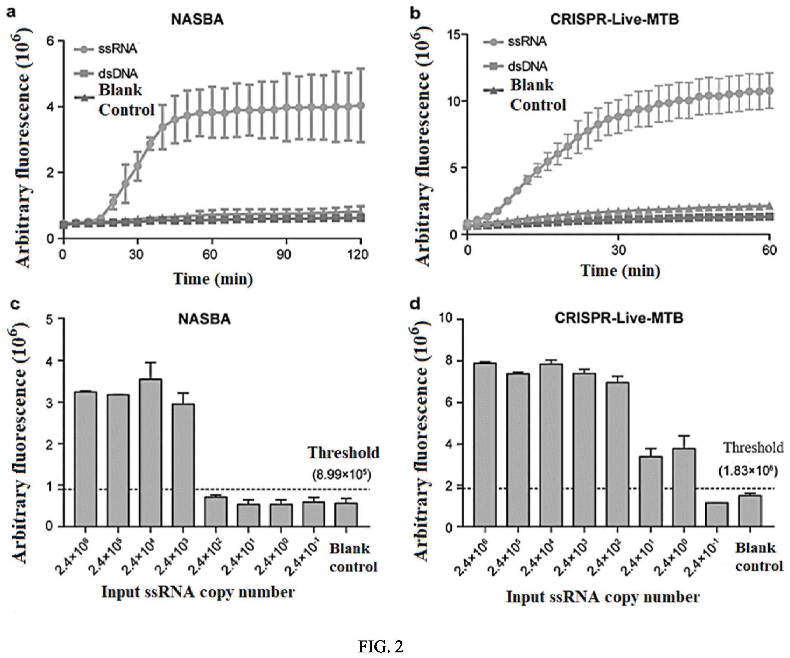

FIG. 2 compares the specific detection of MTB RNA by NASBA and the NASBA-based CRISPR-Live-MTB method; a-b show the specificity of NASBA for the detection of MTB RNA and DNA: a for CRISPR-Live-MTB; b for the input concentration of RNA and DNA templates in the above two reactions ranging from 10⁷ to 10⁸ copies. The NASBA method involved a continuous reaction for 2 hours, while the CRISPR-Live-MTB method involved a 1-hour NASBA reaction followed by a 1-hour CRISPR reaction; c shows the LOD determined by the NASBA method (n = 2 biological replicates; mean ± standard deviation); d shows the LOD determined by the CRISPR-Live-MTB method (n = 3 technical replicates; mean ± standard deviation). The cut-off value for positive signals was set to be higher than the mean of the blank control group ± 3 standard deviations.

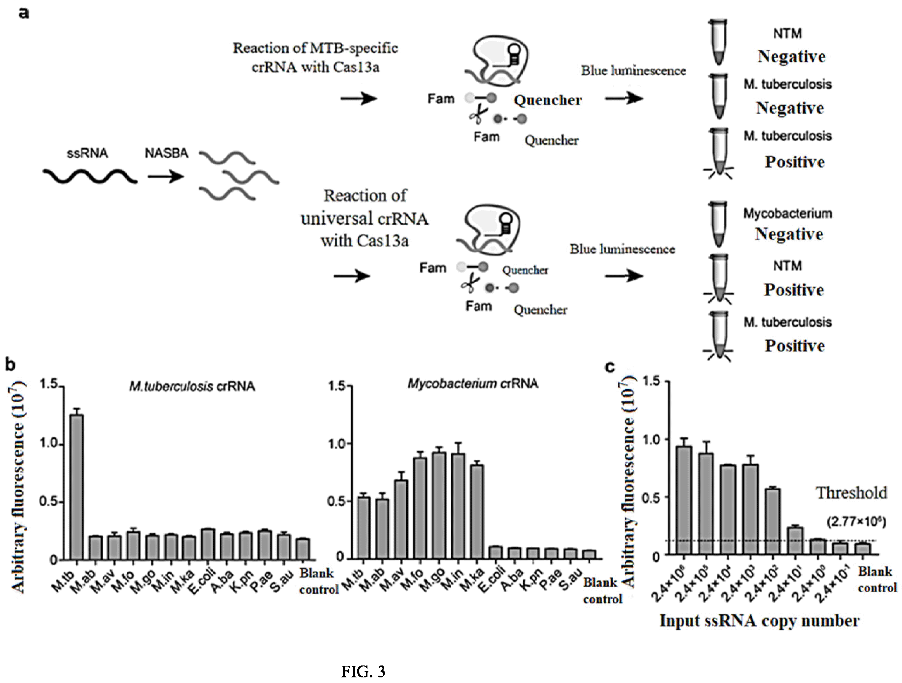

FIG. 3 shows the interspecies reactivity of MTB-specific and universal crRNAs; a: Schematic diagram of the detection of mycobacteria using species-specific or universal crRNA; b: Results of the reaction of MTB-specific crRNA (taking MTB-crRNA5 as an example) and universal mycobacterial crRNA with Cas13a; c: LOD for the detection of mycobacteria determined using universal crRNA and MTB RNA template. Data from the three technical replicates are presented as mean ± standard deviation. The cut-off value for positive signals was set to be equal to the mean of the blank control group ± 3 standard deviations.

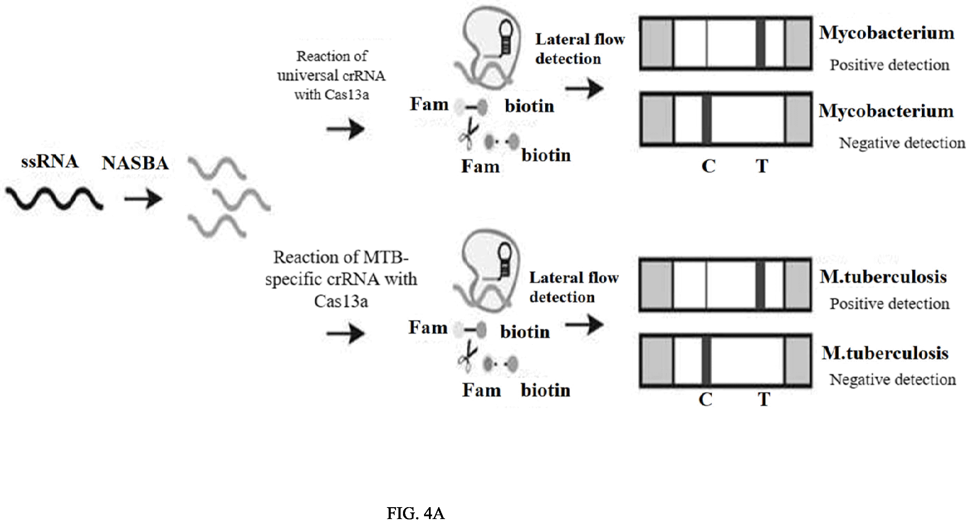

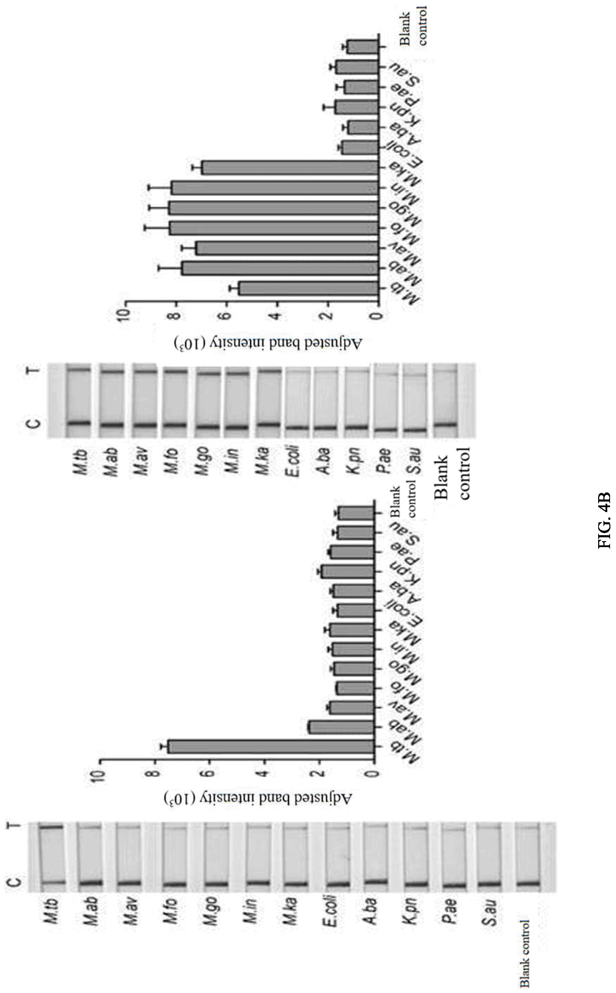

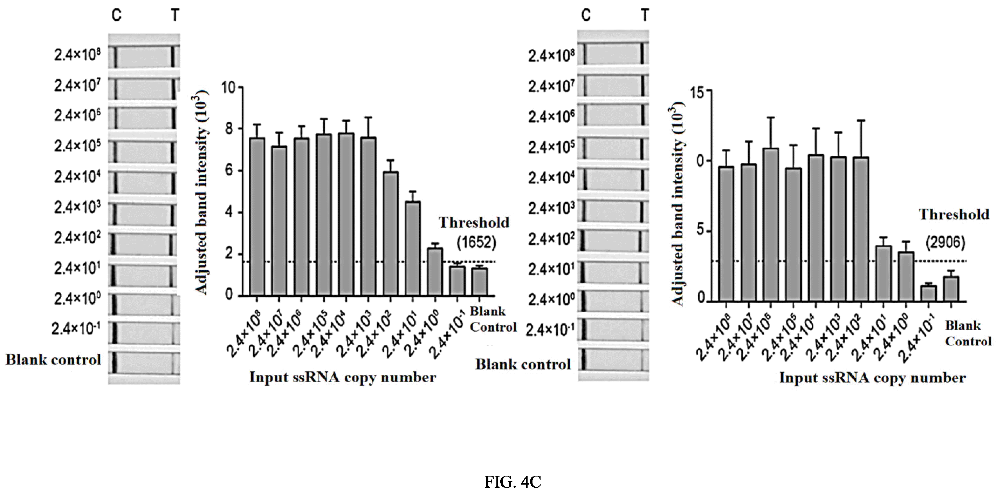

FIG. 4A-FIG. 4C show the lateral flow-based CRISPR-Live-MTB for the detection of MTB and NTM. FIG. 4A is a schematic diagram of the design of lateral flow-based CRISPR-Live-MTB. The left panel of FIG. 4B shows the results of MTB-specific detection using MTB-crRNA5, where the left is the image of the lateral flow strip, and the right is the quantification of band density of the strip. The right panel of FIG. 4B shows the results of Mycobacterium detection using universal crRNA, where the left is the image of the lateral flow strip, and the right is the quantification of band density of the strip. The left panel of FIG. 4C shows the LOD for the detection of MTB RNA using MTB-crRNA5, where the left is the image of the lateral flow strip, and the right is the quantification of band density of the strip. The right panel of FIG. 4C shows the LOD for the detection of MTB RNA using universal crRNA, where the left is the image of the lateral flow strip, and the right is the quantification of band density of the strip. Data from three technical replicates are presented as mean ± standard deviation. The cut-off value for positive signals was set to be equal to the mean of the blank control group ± 3 standard deviations.

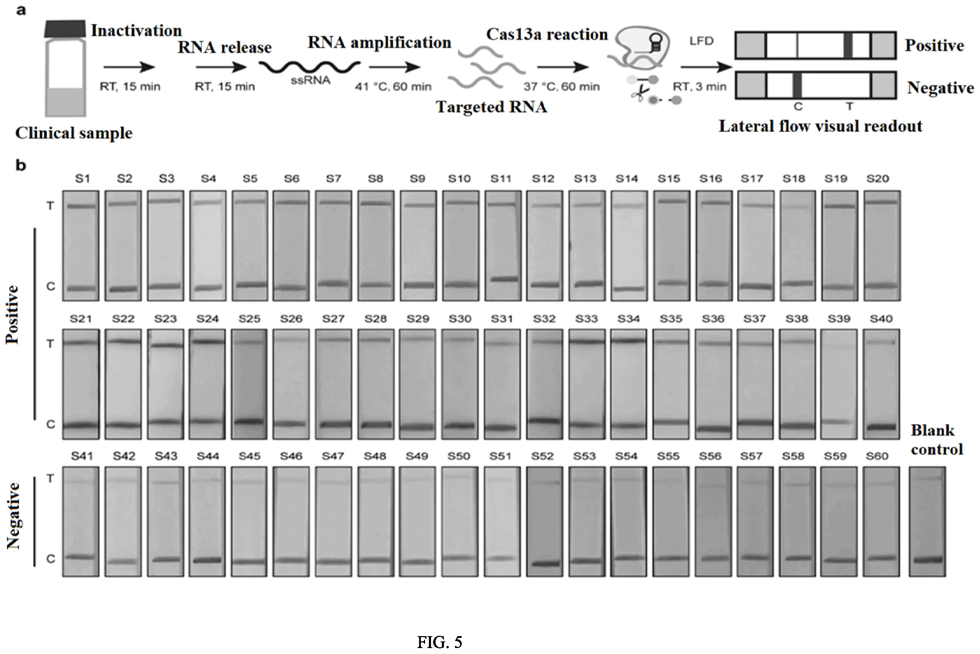

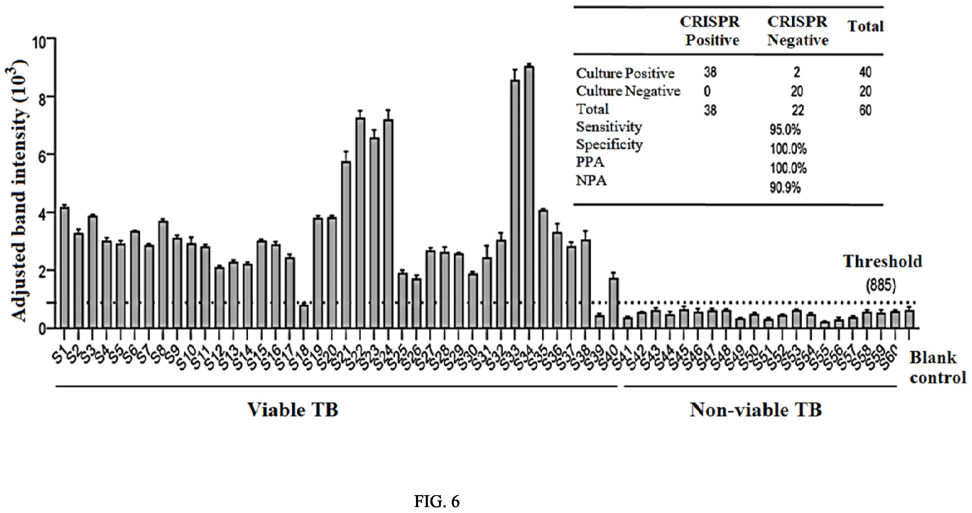

FIG. 5 and FIG. 6 are schematic diagrams of the detection of viable MTB in clinical samples using lateral flow-based CRISPR-Live-MTB. The left panel of FIG. 5 is a schematic diagram of RNA extraction-free CRISPR-Live-MTB, RT: room temperature, LFD: lateral flow detection. The right panel of FIG. 5 shows the results of CRISPR-Live-MTB detection using lateral flow strips. The control band (C) is shown at the bottom, and the test band (T) is shown at the top. Imaging was performed 3 min after sample addition. FIG. 6 shows the quantification of band density in (b). Data from three technical replicates are presented as mean ± standard deviation. The cut-off value for positive signals was set to be equal to the mean of the blank control group± 3 standard deviations. PPA: positive predictive agreement, NPA: negative predictive agreement.

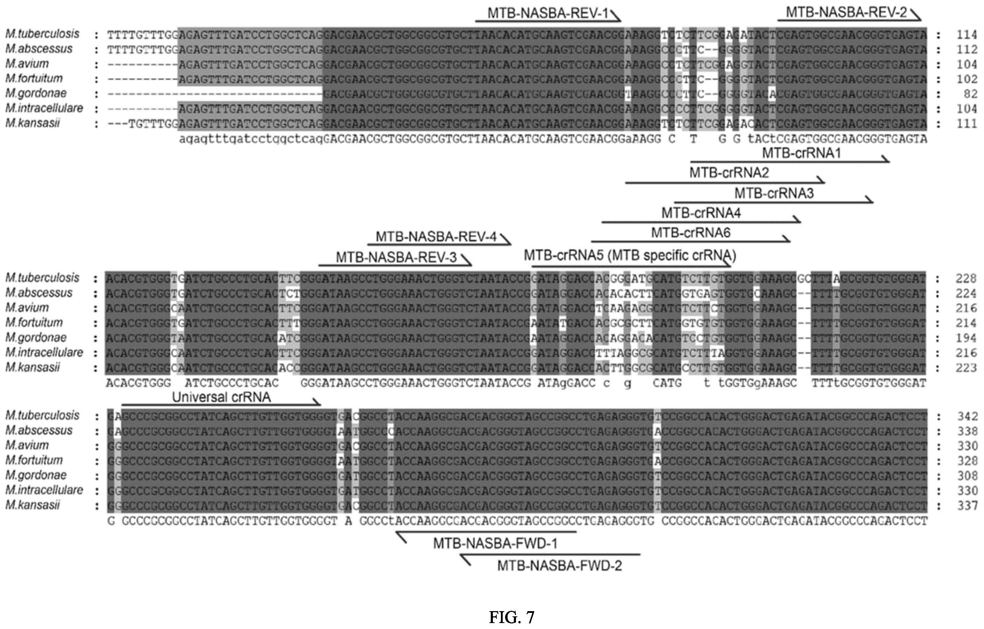

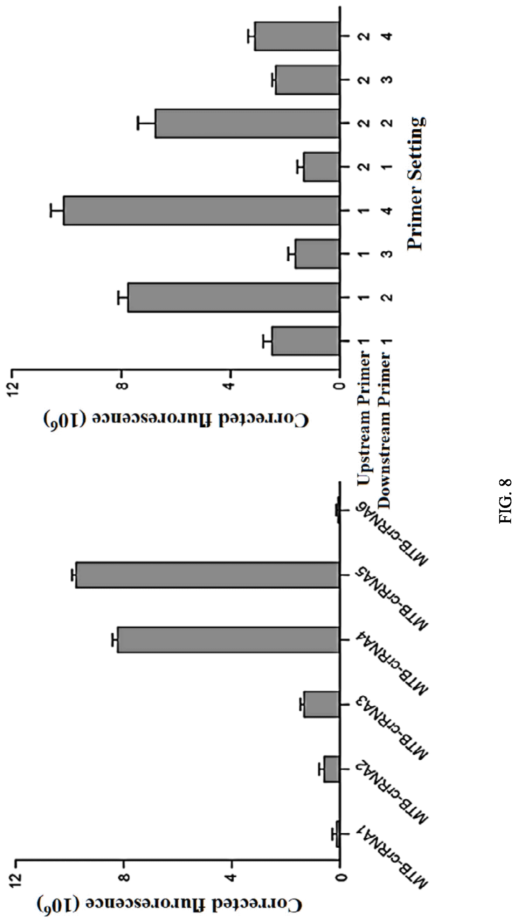

FIG. 7-FIG. 8 are schematic diagrams of the design of crRNAs and NASBA primers for the detection of viable MTB. FIG. 7 shows the sequence alignment of 16S rRNA from MTB and 6 non-tuberculous mycobacteria (NTM), with the binding sites of NASBA primers and Cas13a crRNAs indicated. The lower panel of FIG. 8 shows the screening of crRNAs for NASBA-coupled MTB ssRNA detection, using 1 nM ssRNA template and MTB-NASBA-FWD-1 and MTB-NASBA-REV-2 primers. The upper panel of FIG. 8 shows the screening of NASBA primer combinations for Cas13a cleavage-coupled MTB ssRNA detection, using 1 nM ssRNA template and MTB-crRNA5. In FIG. 8, data from three technical replicates are presented as mean ± standard deviation. Background fluorescence has been removed from each group.

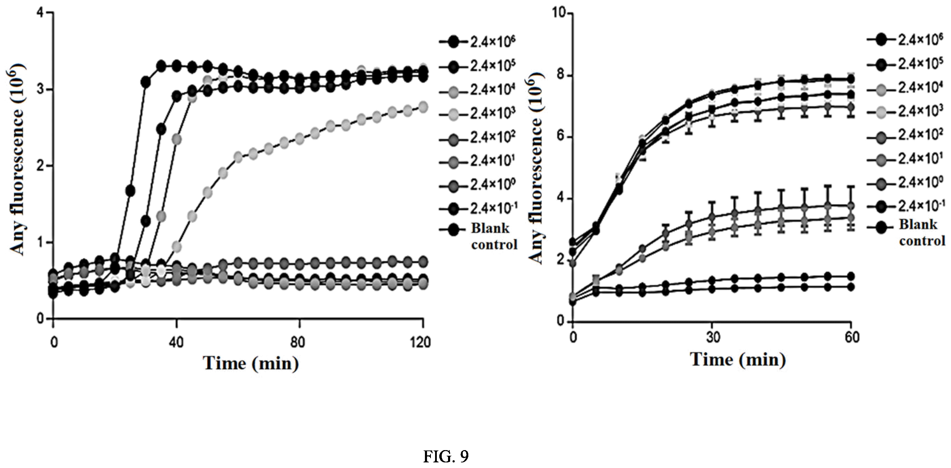

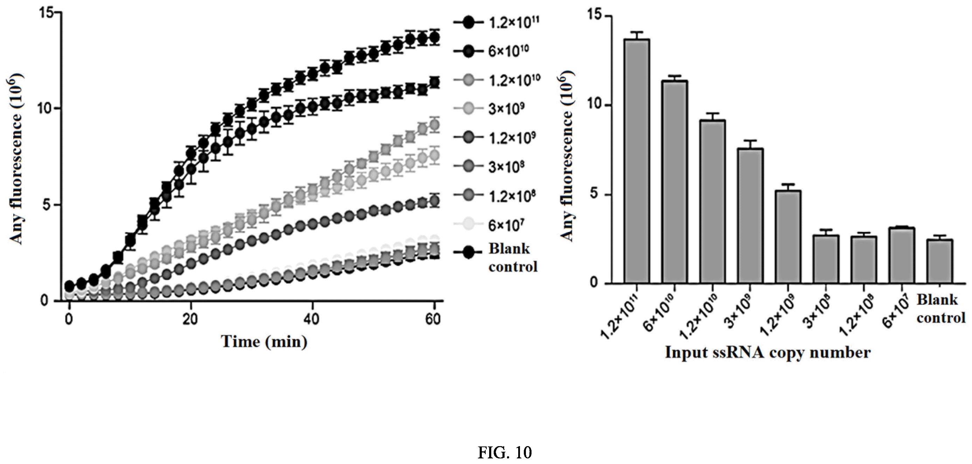

FIG. 9-FIG. 10 show the kinetics of fluorescent signal output of NASBA and CRISPR-Live-MTB; FIG. 9 shows the LOD determination of NASBA (a) and CRISPR-Live-MTB (b), corresponding to c-d of FIG. 2. The lower panel of FIG. 10 shows the NASBA-coupled Cas13a reaction without RNA amplification; the upper panel of FIG. 10 shows the 60-min end-point fluorescence signal of the channel c. Data from three technical replicates are presented as mean ± standard deviation.

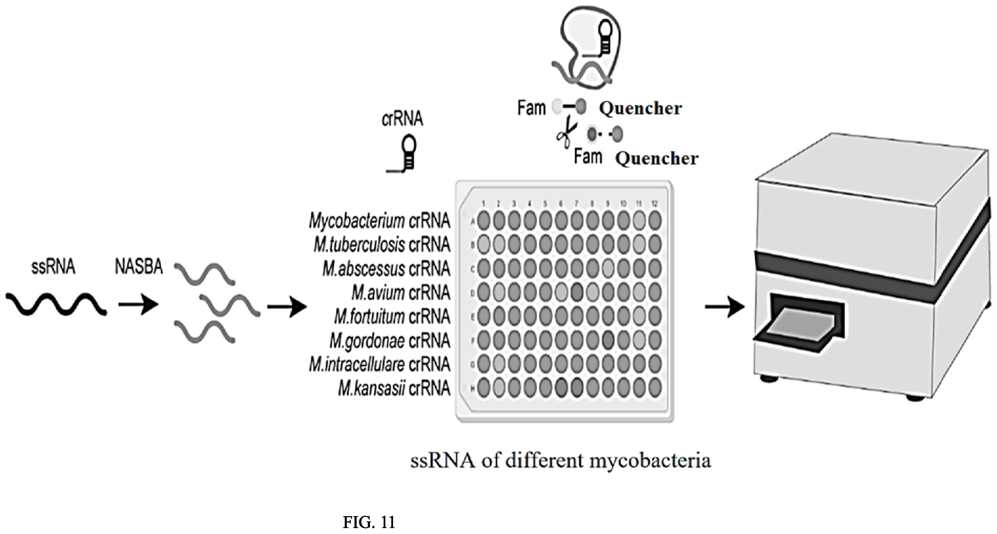

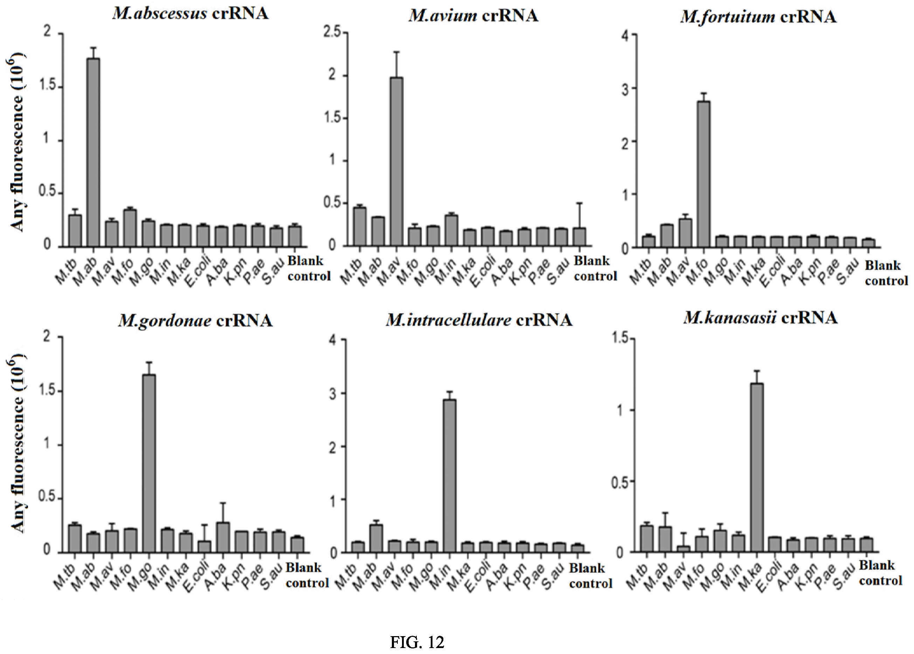

FIG. 11-FIG. 12 are schematic diagrams of species-specific detection of MTB and NTM using the CRISPR-Live-MTB platform; FIG. 11 is a schematic diagram of evaluating species-specific crRNA; FIG. 12 is a schematic diagram of evaluating the interspecific cross-reactivity of species-specific crRNA, with synthesized mycobacterial RNA used as a template; abbreviations are as follows: *M. tb: M. tuberculosis* H37Rv, *M. ab: M. abscessus, M. av: M. avium, M. fo*: *M. fortuitum, M. go: M. gordonae, M. in: M. intracellulare, M. ka: M. kansasii, E. coli: Escherichia coli, A. ba: A. baumannii, K. pn: K. pneumoniae, P. ae: P. aeruginosa, S. au: Staphylococcus aureus.* Data are presented as mean ± standard deviation (n = 3 technical replicates).

### DETAILED DESCRIPTION OF THE PREFERRED EMBODIMENT

The present disclosure is further illustrated below by way of examples, but the present disclosure is not limited to the scope of the examples described herein. Experimental methods without specific conditions indicated in the following examples are selected according to conventional methods and conditions, or according to the product's instructions.

### Example 1

### 1. Materials and Methods

### (1) Strains and Clinical Samples

*Mycobacterium tuberculosis* H37Rv (ATCC 27294) was deposited at the Second Affiliated Hospital of Fujian Medical University. *M. tuberculosis* was cultured in Middlebrook 7H9 broth medium at 37°C.

Collected clinical samples did not contain any personal information and were used solely for molecular diagnostic purposes.

### (2) Sample Processing and RNA Preparation

Samples were processed using the N-acetyl-L-cysteine (NALC)-NaOH method. Approximately 5 mL of sputum was thoroughly mixed with an equal volume of NALC-NaOH solution for inactivation and incubated at room temperature for 15 min. The resulting precipitate was washed once with sterile 0.9% NaCl solution and resuspended in 1. 5 mL of sterile 0.9% NaCl solution. 500 µL of the processed sputum was transferred to a new sterile, nuclease-free 1.5 mL tube, centrifuged at 10,000 g for 5 min. The precipitate was resuspended in 50 µL of lysis buffer (10 mM sodium citrate, pH 8.0) and vortexed. Bacterial cells in the resuspended precipitate were lysed using a water bath sonicator (Shanghai Shengyan Ultrasonic Instrument Co., Ltd.) at 300 W for 15 min at room temperature, followed by centrifugation at 10,000 g for 5 min. The obtained supernatant was used as the template for subsequent experiments.

### (3) Expression and Purification of LwCasl3a Protein

Cas13a (LwCas13a) protein from *Leptotrichia wadei* was purified with minor modifications compared to the method described in Gootenberg JS, Abudayyeh OO, Lee JW, et al. Nucleic acid detection with CRISPR-Cas13a/C2c2. Science.2017 Apr 28; 356(6336):438-442. The codon-optimized LwCas13a coding gene (SEQ ID NO: 23) was cloned into the bacterial expression vector pET28a. A 6×His tag (positions 3478-3495 of SEQ ID NO: 23) was added to the N-terminus of LwCas13a, and its expression was controlled by the T7 promoter (SEQ ID NO: 24). The LwCas13a expression vector was transformed into *Escherichia coli* BL21 (DE3). Fresh clones were picked and cultured overnight in Luria-Bertani (LB) medium supplemented with 50 µg/mL kanamycin at 37°C with shaking. The culture (10 mL) was inoculated into 2 L of LB medium containing 50 µg/mL kanamycin, and cultured at 37°C with shaking at 220 rpm. When the optical density at 600 nm (OD₆₀₀) reached 0.6-0.8, isopropyl β-D-1-thiogalactopyranoside (IPTG) was added to the culture to a final concentration of 0.5 mM. The culture was incubated overnight at 16°C with shaking to induce protein expression. Cells were harvested by centrifugation at 5,200 g for 15 min at 4°C and stored at -80°C.

For protein purification, cells were resuspended in lysis buffer (20 mM Tris-HCl, 500 mM NaCl, 1 mM DTT, pH 8.0) and sonicated on ice for 10 min under the following conditions: amplitude 100, 5 s on and 5 s off per cycle. The resulting debris was removed by centrifugation at 10,000 g for 1 h at 4°C, and the supernatant was filtered through a Stericup 0.22 µm filter membrane (EMD Millipore). The filtered supernatant was loaded onto a Ni-NTA column (Qiagen) for affinity purification. Further purification was performed using fast protein liquid chromatography (FPLC) with a Superdex 200 size exclusion column (GE Healthcare Life Sciences). The purified protein was analyzed by SDS-PAGE, buffer exchanged into storage buffer (50 mM Tris-HCl, pH 7.5, 600 mM NaCl, 5 % (v/v) glycerol, 2 mM dithiothreitol (DTT)), and frozen at -80°C.

### (4) NASBA Primer Design and crRNA Preparation

As shown in FIG. 7, the 16S rRNA sequence of MTB H37Rv (ATCC 27294) and sequences of 6 other reference strains were obtained from the NCBI database (MTB H37Rv (ATCC 27294): SEQ ID NO: 25; *M. abscessus* (ATCC 19977): SEQ ID NO: 26; *M. avium* (ATCC 25291): SEQ ID NO: 27; *M. fortuitum* (ATCC 6841): SEQ ID NO: 28; *M. gordonae* (ATCC 14470): SEQ ID NO: 29; *M. intracellulare* (ATCC 13950): SEQ ID NO: 30; *M. kansasii* (ATCC 12478): SEQ ID NO: 31; the lower sequence: SEQ ID NO: 32) and aligned using MEGAX to identify conserved and variable regions. NASBA primers were designed against the conserved nucleotide regions of the 16S rRNA gene. A T7 promoter sequence was linked to the 5' end of the NASBA upstream primer. Primer sequences are shown in Table 1 and were synthesized by GENEWIZ.

DNA templates for crRNA, including the linked T7 promoter sequence, were synthesized using GENEWIZ's primers. The crRNA templates were annealed to form a short T7 primer (T7-3G), followed by overnight transcription of crRNA at 37°C using the HiScribe T7 Quick High Yield RNA Synthesis Kit. The crRNA was purified using RNAiso Plus (Takara, 9108) according to the manufacturer's instructions. The purified crRNA was stored at -80°C.

**Table 1 Primers used in the examples**

| **Name** | **SEQ ID NO** | **Sequence (5' to 3')*** |
|---|---|---|
| MTB-NASBA-FWD-1 | 1 | aattc**TAATACGACTCACTATAGGG**gccg gctacccgtcgtcgccttggt |
| MTB-NASBA-FWD-2 | 2 | aattcTAATACGACTCACTATAGGGccct ctcaggccggctacccgtcgtc |
| MTB-NASBA-REV-1 | 3 | taacacatgcaagtcgaacg |
| MTB-NASBA-REV-2 | 4 | cgagtggcgaacgggtgagt |
| MTB-NASBA-REV-3 | 5 | gataagcctgggaaactgggtc |
| MTB-NASBA-REV-4 | 6 | ctgggaaactgggtctaatac |
| Taqman probe for fluorescent NASBA reaction | 7 | 6FAM-ccagcggauaggaccacggcugg- DABCYL |
| T7-3G | 8 | gaaattaatacgactcactataggg |

Underlined sequences are target binding regions; bold uppercase sequences are the T7 promoter sequence.

T7 promoter sequence (SEQ ID NO: 24):
TAATACGACTCACTATAGGG

### (5) RT-PCR

2 µL of RNA sample was reverse transcribed into cDNA using the PrimeScript RT reagent Kit (Takara). PCR primers are shown in Table 1. The 10 µL reaction system contained 0.5 µL of reverse-transcribed cDNA product, 5 µL of PowerUp SYBR Green Master Mix (Applied Biosystems), 0.5 µL of each forward and downstream primer (10 µM stock solution), mixed at 4°C and incubated in a QuantStudio 6 Flex System thermocycler (Applied Biosystems). RT-PCR reaction conditions were as follows: 50°C for 2 min, 95°C for 2 min, followed by cycles of 95°C denaturation for 15 s and 60°C annealing/extension for 1 min. Fluorescence signals were collected at the 60°C annealing/extension step of each cycle.

### (6) RNA-Specific Isothermal Amplification by NASBA

RNA-specific isothermal amplification was performed using a commercial NASBA kit (NECB-24, Life Sciences) according to the manufacturer's instructions. Briefly, each 20 µL reaction system contained 6.7 µL of reaction buffer (Life Sciences, NECB-24), 3.3 µL of Nucleotide Mix (Life Sciences, NECN-24), 0.5 µL of nuclease-free water, 0.4 µL of 12. 5 µM NASBA primers, 0.1 µL of Murine RNase inhibitor (Vazyme, R301-01), and 4 µL of RNA (water as the negative control). The above components were mixed at 4°C, incubated at 65°C for 2 min, and then at 41°C for 10 min. Subsequently, 5 µL of enzyme mix (Life Sciences, NEC-1-24) was added to each reaction system, and the reaction mixture was incubated at 41°C for 1 to 2 h. Fluorescent NASBA was performed by adding 50 nM of RNA probe (as shown in Table 1) to the 20 mL reaction system. The 5' end of the RNA probe was labeled with 6-carboxyfluorescein (FAM) phosphoramidite, and the 3' end was labeled with 4-[4-(dimethylamino)phenylazo] benzoic acid N-succinimidyl ester (DABCYL). The fluorescence signal of NASBA was detected using a SpectraMax iD3 Multi-Mode Microplate Reader (Molecular Devices) with an excitation wavelength of 485 nm and an emission wavelength of 520 nm.

### (7) LwCas13a Reaction

LwCasl3a detection assay was performed as previously described. The 20 µL reaction system contained 50 nM of purified LwCas13a, 0.25 µM of FAM-BQ1-labeled ssRNA probe (Genescript), 10 ng of crRNA (as shown in Table 2), 6 mM MgCl2, 20 mM Tris-HCl, pH 7. 4, 0.1 µL of Murine RNase inhibitor (Vazyme, R301-01), and 3 µL of NASBA product. The reaction mixture was incubated at 37°C for 1 h, and fluorescence signals were collected using a SpectraMax iD3 Multi-Mode Microplate Reader with an excitation wavelength of 485 nm and an emission wavelength of 520 nm. Fluorescence kinetics were measured every 5 min. For lateral flow-based detection, commercially available lateral flow test strips (Milenia HybriDetect 1, TwistDx) were used according to the manufacturer's instructions. The FAM-BQ1-labeled ssRNA probe was replaced with a FAM-biotin-labeled ssRNA probe, and the final concentration was adjusted to 1 µM in 20 µL of LwCas13a reaction solution. The LwCas13a reaction was performed at 37°C for 1 h, then 20 µL of the product was transferred to 100 µL of Hybridetect assay buffer and applied to the lateral flow strip. The band intensity of the strip can be read directly with the naked eye or imaged with a camera for further density analysis.

**Table 2 crRNAs used in the examples**

| **Name** | **SEQ ID NO** | **Full crRNA sequence** |
|---|---|---|
| MTB-crRNA1 | 9 | ggggauuuagacuaccccaaaaacgaaggggacuaaaaccuugug guggaaagcgcuuuagcggugu |
| MTB-crRNA2 | 10 | ggggauuuagacuaccccaaaaacgaaggggacuaaaaccauguc uugugguggaaagcgcuuuagc |
| MTB-crRNA3 | 11 | ggggauuuagacuaccccaaaaacgaaggggacuaaaacggugga aagcgcuuuagcggugugggau |
| MTB-crRNA4 | 12 | ggggauuuagacuaccccaaaaacgaaggggacuaaaaccgggau gcaugucuugugguggaaagcg |
| MTB-crRNA5 | 13 | ggggauuuagacuaccccaaaaacgaaggggacuaaaacgauagg accacgggaugcaugucuugug |
| MTB-crRNA6 | 14 | ggggauuuagacuaccccaaaaacgaaggggacuaaaacaccgga uaggaccacgggaugcaugucu |
| Universal-crRNA | 15 | ggggauuuagacuaccccaaaaacgaaggggacuaaaacgcccgc ggccuaucagcuuguuggugggg |
| *M. gordonae* detection crRNA | 16 | ggggauuuagacuaccccaaaaacgaaggggacuaaaacaauagg accacaggacacauguccugug |
| *M. abscessus* detection crRNA | 17 | ggggauuuagacuaccccaaaaacgaaggggacuaaaacgauagg accacacacuucauggugagug |
| *M. fortuitum* detection crRNA | 18 | ggggauuuagacuaccccaaaaacgaaggggacuaaaacaauaug accacgcgcuucauggugugug |
| *M. avium* detection crRNA | 19 | ggggauuuagacuaccccaaaaacgaaggggacuaaaacgauagg accucaagacgcaugucuucug |
| *M. intracellulare* detection crRNA | 20 | ggggauuuagacuaccccaaaaacgaaggggacuaaaacgauagg accuuuaggcgcaugucuuuag |
| *M. kansasii* detection crRNA | 21 | ggggauuuagacuaccccaaaaacgaaggggacuaaaacgauagg accacuuggcgcaugccuugug |

Underlined sequences are spacer sequences.

LwCasl3a construct sequence used in the examples (SEQ ID NO: 22):

MGSSHHHHHHSSGLVPRGSMKVTKVDGISHKKYIEEGKLVKSTSEENRTSER
LSELLSIRLDIYIKNPDNASEEENRIRRENLKKFFSNKVLHLKDSVLYLKNRKEKNAVQ
DKNYSEEDISEYDLKNKNSFSVLKKILLNEDVNSEELEIFRKDVEAKLNKINSLKYSFE
ENKANYQKINENNVEKVGGKSKRNIIYDYYRESAKRNDYINNVQEAFDKLYKKEDIE
KLFFLIENSKKHEKYKIREYYHKIIGRKNDKENFAKIIYEEIQNVNNIKELIEKIPDMSE
LKKSQVFYKYYLDKEELNDKNIKYAFCHFVEIEMSQLLKNYVYKRLSNISNDKIKRIF
EYQNLKKLIENKLLNKLDTYVRNCGKYNYYLQVGEIATSDFIARNRQNEAFLRNIIGV
SSVAYFSLRNILETENENDITGRMRGKTVKNNKGEEKYVSGEVDKIYNENKQNEVKE
NLKMFYSYDFNMDNKNEIEDFFANIDEAISSIRHGIVHFNLELEGKDIFAFKNIAPSEIS
KKMFQNEINEKKLKLKIFKQLNSANVFNYYEKDVIIKYLKNTKFNFVNKNIPFVPSFT
KLYNKIEDLRNTLKFFWSVPKDKEEKDAQIYLLKNIYYGEFLNKFVKNSKVFFKITNE
VIKINKQRNQKTGHYKYQKFENIEKTVPVEYLAIIQSREMINNQDKEEKNTYIDFIQQI
FLKGFIDYLNKNNLKYIESNNNNDNNDIFSKIKIKKDNKEKYDKILKNYEKHNRNKEI
PHEINEFVREIKLGKILKYTENLNMFYLILKLLNHKELTNLKGSLEKYQSANKEETFSD
ELELINLLNLDNNRVTEDFELEANEIGKFLDFNENKIKDRKELKKFDTNKIYFDGENII
KHRAFYNIKKYGMLNLLEKIADKAKYKISLKELKEYSNKKNEIEKNYTMQQNLHRK
YARPKKDEKFNDEDYKEYEKAIGNIQKYTHLKNKVEFNELNLLQGLLLKILHRLVGY
TSIWERDLRFRLKGEFPENHYIEEIFNFDNSKNVKYKSGQIVEKYINFYKELYKDNVE
KRSIYSDKKVKKLKQEKKDLYIRNYIAHFNYIPHAEISLLEVLENLRKLLSYDRKLKN
AIMKSIVDILKEYGFVATFKIGADKKIEIQTLESEKIVHLKNLKKKKLMTDRNSEELCE
LVKVMFEYKALEKLAAALEHHHHHH

Codon-optimized LwCas13a sequence (SEQ ID NO: 23) used in the examples:

atgaaagtgaccaaggtcgacggcatcagccacaagaagtacatcgaagagggcaagctcgtgaagtccaccagcga
ggaaaaccggaccagcgagagactgagcgagctgctgagcatccggctggacatctacatcaagaaccccgacaacgcctccgag
gaagagaaccggatcagaagagagaacctgaagaagttctttagcaacaaggtgctgcacctgaaggacagcgtgctgtatctgaag
aaccggaaagaaaagaacgccgtgcaggacaagaactatagcgaagaggacatcagcgagtacgacctgaaaaacaagaacagc

ttctccgtgctgaagaagatcctgctgaacgaggacgtgaactctgaggaactggaaatctttcggaaggacgtggaagccaagctga
acaagatcaacagcctgaagtacagcttcgaagagaacaaggccaactaccagaagatcaacgagaacaacgtggaaaaagtggg
cggcaagagcaagcggaacatcatctacgactactacagagagagcgccaagcgcaacgactacatcaacaacgtgcaggaagcc
ttcgacaagctgtataagaaagaggatatcgagaaactgtttttcctgatcgagaacagcaagaagcacgagaagtacaagatccgcg
agtactatcacaagatcatcggccggaagaacgacaaagagaacttcgccaagattatctacgaagagatccagaacgtgaacaaca
tcaaagagctgattgagaagatccccgacatgtctgagctgaagaaaagccaggtgttctacaagtactacctggacaaagaggaact
gaacgacaagaatattaagtacgccttctgccacttcgtggaaatcgagatgtcccagctgctgaaaaactacgtgtacaagcggctga
gcaacatcagcaacgataagatcaagcggatcttcgagtaccagaatctgaaaaagctgatcgaaaacaaactgctgaacaagctgg
acacctacgtgcggaactgcggcaagtacaactactatctgcaagtgggcgagatcgccacctccgactttatcgcccggaaccggc
agaacgaggccttcctgagaaacatcatcggcgtgtccagcgtggcctacttcagcctgaggaacatcctggaaaccgagaacgag
aacgatatcaccggccggatgcggggcaagaccgtgaagaacaacaagggcgaagagaaatacgtgtccggcgaggtggacaa
gatctacaatgagaacaagcagaacgaagtgaaagaaaatctgaagatgttctacagctacgacttcaacatggacaacaagaacga
gatcgaggacttcttcgccaacatcgacgaggccatcagcagcatcagacacggcatcgtgcacttcaacctggaactggaaggcaa
ggacatcttcgccttcaagaatatcgcccccagcgagatctccaagaagatgtttcagaacgaaatcaacgaaaagaagctgaagctg
aaaatcttcaagcagctgaacagcgccaacgtgttcaactactacgagaaggatgtgatcatcaagtacctgaagaataccaagttcaa
cttcgtgaacaaaaacatccccttcgtgcccagcttcaccaagctgtacaacaagattgaggacctgcggaataccctgaagtttttttgg
agcgtgcccaaggacaaagaagagaaggacgcccagatctacctgctgaagaatatctactacggcgagttcctgaacaagttcgtg
aaaaactccaaggtgttctttaagatcaccaatgaagtgatcaagattaacaagcagcggaaccagaaaaccggccactacaagtatc
agaagttcgagaacatcgagaaaaccgtgcccgtggaatacctggccatcatccagagcagagagatgatcaacaaccaggacaaa
gaggaaaagaatacctacatcgactttattcagcagattttcctgaagggcttcatcgactacctgaacaagaacaatctgaagtatatcg
agagcaacaacaacaatgacaacaacgacatcttctccaagatcaagatcaaaaaggataacaaagagaagtacgacaagatcctga
agaactatgagaagcacaatcggaacaaagaaatccctcacgagatcaatgagttcgtgcgcgagatcaagctggggaagattctga
agtacaccgagaatctgaacatgttttacctgatcctgaagctgctgaaccacaaagagctgaccaacctgaagggcagcctggaaaa
gtaccagtccgccaacaaagaagaaaccttcagcgacgagctggaactgatcaacctgctgaacctggacaacaacagagtgaccg
aggacttcgagctggaagccaacgagatcggcaagttcctggacttcaacgaaaacaaaatcaaggaccggaaagagctgaaaaa
gttcgacaccaacaagatctatttcgacggcgagaacatcatcaagcaccgggccttctacaatatcaagaaatacggcatgctgaatc
tgctggaaaagatcgccgataaggccaagtataagatcagcctgaaagaactgaaagagtacagcaacaagaagaatgagattgaa
aagaactacaccatgcagcagaacctgcaccggaagtacgccagacccaagaaggacgaaaagttcaacgacgaggactacaaa
gagtatgagaaggccatcggcaacatccagaagtacacccacctgaagaacaaggtggaattcaatgagctgaacctgctgcaggg
cctgctgctgaagatcctgcaccggctcgtgggctacaccagcatctgggagcgggacctgagattccggctgaagggcgagtttcc

cgagaaccactacatcgaggaaattttcaatttcgacaactccaagaatgtgaagtacaaaagcggccagatcgtggaaaagtatatca
acttctacaaagaactgtacaaggacaatgtggaaaagcggagcatctactccgacaagaaagtgaagaaactgaagcaggaaaaa
aaggacctgtacatccggaactacattgcccacttcaactacatcccccacgccgagattagcctgctggaagtgctggaaaacctgc
ggaagctgctgtcctacgaccggaagctgaagaacgccatcatgaagtccatcgtggacattctgaaagaatacggcttcgtggccac
cttcaagatcggcgctgacaagaagatcgaaatccagaccctggaatcagagaagatcgtgcacctgaagaatctgaagaaaaaga
aactgatgaccgaccggaacagcgaggaactgtgcgaactcgtgaaagtcatgttcgagtacaaggccctggaaaagcttgcggcc
gcactcgagcaccaccaccaccaccactga

### 2. Results

As shown in FIG. 1, in the NASBA reaction, MTB rRNA is first reverse transcribed into complementary DNA (cDNA), and then the rRNA in the RNA-DNA duplex is degraded by RNase H. Double-stranded DNA (dsDNA) is synthesized using the cDNA as the template. Through the T7 transcription reaction, the newly synthesized dsDNA produces RNA products. Importantly, in this process, MTB genomic DNA no longer serves as a template for RNA production due to the absence of the T7 promoter. Therefore, the NASBA reaction of the present disclosure enables RNA-dependent amplification of MTB. The products of the NASBA reaction are used for the Cas13a reaction, which induces the collateral cleavage of the ssRNA reporter for subsequent fluorescence analysis or lateral flow analysis.

As shown in FIG. 2 and FIG. 8, comparative analysis indicated that MTB-crRNA5 generated the strongest fluorescent signal. Unless otherwise specified, subsequent experiments in the examples were based on MTB-crRNA5.

In addition, NASBA amplification plays a critical role in fluorescent signal output. The inventors designed two upstream primers and four downstream primers, as well as combinations thereof, to quantify the amplification efficiency via the fluorescent signal of the Cas13a reaction with MTB-crRNA5. The results showed that the combination of MTB-NASBA-FWD-1 and MTB-NASBA-REV-4 had the highest efficiency.

The example compared the sensitivity and specificity of detecting MTB RNA relative to DNA. The total reaction time was fixed at 2 h, where the NASBA method included a continuous 2-h reaction, and the CRISPR-Live-MTB method included a 1-h NASBA reaction followed by a 1-h CRISPR reaction. The results showed that both NASBA and CRISPR-Live-MTB exhibited higher fluorescence intensity when using ssRNA as the template than when using DNA as the template, with CRISPR-Live-MTB showing a higher fluorescence signal intensity.

Subsequently, the inventors compared the limit of detection (LOD) of the fluorescent NASBA reaction in CRISPR-Live-MTB. As shown in FIG. 9-FIG. 10, the results indicated that the LOD of the 2-h NASBA reaction was 2,400 copies (corresponding to a template concentration of 0.2 fM); the LOD of CRISPR-Live-MTB (1-h NASBA reaction + 1-h CRISPR reaction) was only 2.4 copies (corresponding to a template concentration of 0.2 aM), demonstrating the critical role of the CRISPR reaction in CRISPR-Live-MTB.

As shown in FIG. 3, crRNA screening indicated that MTB-crRNA5 had higher specificity for MTB compared with NTM and non-mycobacterial bacteria. In addition, as shown in FIG. 11-FIG. 12, the inventors also designed a universal crRNA for Mycobacterium genus based on the conserved genomic sequences of MTB and NTM, which exhibited broad activity against all tested Mycobacterium species, including MTB and NTM, while retaining specificity against unrelated bacteria. The LOD of the universal crRNA was determined to be 2.4 copies (corresponding to a template concentration of 0.2 aM) when using MTB RNA as the template. The single-digit LOD values, which were comparable between MTB-crRNA5 and the universal mycobacterial crRNA, demonstrate that the CRISPR-Live-MTB of the present disclosure is a highly sensitive diagnostic platform.

To explore the application of CRISPR-Live-MTB in POCT, a lateral flow-based signal readout method was designed. In this method, the reporter molecule in the fluorescent matrix was replaced by FAM-biotin-labeled ssRNA, enabling the cleavage signal to be visualized on commercially available lateral flow strips. As shown in FIG. 4A-FIG. 4C, the control line is displayed based on the interaction between immobilized streptavidin and flow-through biotin. In the presence of MTB RNA, the ssRNA is cleaved, releasing free FAM molecules, which can be captured by gold nanoparticle-conjugated anti-FAM antibodies to display the test line.

The results showed that the LOD of both MTB-crRNA5 and the universal mycobacterial crRNA was consistent with that of the fluorescence reaction, at 2.4 copies (corresponding to a template concentration of 0.2 aM). These results indicate that lateral flow-based CRISPR-Live-MTB is rapid, specific and highly sensitive for the detection of viable MTB.

To enable more rapid detection of MTB RNA, the inventors sought to integrate an extraction-free RNA preparation method into CRISPR-Live-MTB. Accordingly, the aforementioned clinical samples were inactivated for 15 min and then sonicated for 15 min by the NALC-NaOH method to release RNA. The obtained RNA samples were subjected to CRISPR-Live-MTB detection, and the results were analyzed using lateral flow strips and a densitometer.

As shown in FIG. 5 and FIG. 6, the detection results indicated that among the 60 samples, 40 samples (S1-S40) were confirmed to contain viable MTB by the culture method, and the other 20 samples (S41-S60) contained no viable MTB. Detection was performed using CRISPR-Live-MTB, with the control group using the mean positive signal threshold plus threefold the standard deviation (mean + 3SD); 38 out of the 40 culture-confirmed positive samples were verified to contain viable MTB, with the remaining 2 samples being S18 and S39, showing a sensitivity of approximately 95.0%. All 20 negative samples (S41-S60) tested negative by CRISPR-Live-MTB, showing a specificity of 100.0%. Overall, CRISPR-Live-MTB achieved a positive detection rate of 100.0% and a negative detection rate of 90.9%.

While specific embodiments of the present disclosure are described above, those skilled in the art should understand that these embodiments are merely illustrative, and various changes or modifications can be made to these embodiments without departing from the principles and essence of the present disclosure. Therefore, the scope of protection of the present disclosure is defined by the appended claims.

## Claims

1. A primer pair for detecting *Mycobacterium tuberculosis,* comprising an upstream primer and a downstream primer;
the upstream primer comprises a nucleotide sequence as shown in SEQ ID NO: 1 or 2, and the downstream primer comprises a nucleotide sequence as shown in any one of SEQ ID NOs: 3-6.

2. The primer pair according to claim 1, wherein the upstream primer and the downstream primer are selected from the following combinations:
(1) the upstream primer comprises a nucleotide sequence as shown in SEQ ID NO: 1, and the downstream primer comprises a nucleotide sequence as shown in SEQ ID NO: 6;
(2) the upstream primer comprises a nucleotide sequence as shown in SEQ ID NO: 1, and the downstream primer comprises a nucleotide sequence as shown in SEQ ID NO: 4;
(3) the upstream primer comprises a nucleotide sequence as shown in SEQ ID NO: 2, and the downstream primer comprises a nucleotide sequence as shown in SEQ ID NO: 4;
(4) the upstream primer comprises a nucleotide sequence as shown in SEQ ID NO: 2, and the downstream primer comprises a nucleotide sequence as shown in SEQ ID NO: 6;
(5) the upstream primer comprises a nucleotide sequence as shown in SEQ ID NO: 1, and the downstream primer comprises a nucleotide sequence as shown in SEQ ID NO: 3;
(6) the upstream primer comprises a nucleotide sequence as shown in SEQ ID NO: 2, and the downstream primer comprises a nucleotide sequence as shown in SEQ ID NO:5;
(7) the upstream primer comprises a nucleotide sequence as shown in SEQ ID NO: 1, and the downstream primer comprises a nucleotide sequence as shown in SEQ ID NO: 5;
(8) the upstream primer comprises a nucleotide sequence as shown in SEQ ID NO: 2, and the downstream primer comprises a nucleotide sequence as shown in SEQ ID NO: 3;
preferably, the 5' end of the upstream primer further comprises a T7 promoter sequence; the sequence of T7 promoter is shown, for example, in SEQ ID NO: 24.

3. A nucleic acid detection kit for nucleic acid-dependent amplification, comprising the primer pair of claim 1 or 2;
preferably, the nucleic acid detection kit further comprises a reverse transcriptase, an RNase inhibitor, and an RNA polymerase;
more preferably, the RNA polymerase is a T7 RNA polymerase; and/or, the nucleic acid detection kit further comprises an RNA template.

4. A reaction system for nucleic acid-dependent amplification, comprising the primer pair of claim 1 or 2;
preferably:
the working concentration of the primer pair is 0.05-1 µM, preferably 0.25-1 µM; and/or, the reaction system further comprises a reverse transcriptase, an RNase inhibitor, and an RNA polymerase.

5. An amplification method for nucleic acid-dependent amplification, comprising adding an RNA template to the reaction system of claim 4 to perform an amplification reaction;
preferably, the RNA template is a 16S rRNA template; and/or, the working concentration of the RNA template is at least 0.2 aM, or 1 to 1000 times lower than the working concentration specified in the instructions of a NASBA kit such as NECB-24; and/or, the temperature of the amplification reaction is 41-42°C.

6. A crRNA for detecting *Mycobacterium,* comprising a spacer sequence of the nucleotide sequence as shown in any one of SEQ ID NOs: 9-21;
preferably, the spacer sequence is a nucleotide sequence of 28 bp in length located at the 3' end of the nucleotide sequence as shown in any one of SEQ ID NOs: 9-21; and/or, the crRNA comprises a spacer sequence of the nucleotide sequence as shown in SEQ ID NO: 15.

7. A crRNA for detecting *Mycobacterium tuberculosis, Mycobacterium gordonae, Mycobacterium abscessus, Mycobacterium fortuitum, Mycobacterium avium, Mycobacterium intracellulare* or *Mycobacterium kansasii,* comprising a spacer sequence of the nucleotide sequence as shown in any one of SEQ ID NOs: 9-14, SEQ ID NO: 16, SEQ ID NO: 17, SEQ ID NO: 18, SEQ ID NO: 19, SEQ ID NO: 20, or SEQ ID NO: 21, respectively;
preferably, the spacer sequence is a nucleotide sequence of 28 bp in length located at the 3' end of the nucleotide sequence.

8. A CRISPR detection kit, comprising the crRNA of claim 6 or 7;
preferably, the CRISPR detection kit further comprises a Cas protein, such as LwaCas13a;
more preferably, the CRISPR detection kit further comprises an ssRNA fluorescent probe, the ssRNA fluorescent probe comprises, for example, a nucleotide sequence as shown in SEQ ID NO: 7.

9. A reaction system for CRISPR detection, comprising the crRNA of claim 6 or 7;
preferably, the working concentration of the crRNA is 0.1-2.5 ng/µL, preferably 0.5-2.5 ng/µL; and/or, the reaction system further comprises a Cas protein, such as LwaCas13a; and/or, the working concentration of the Cas protein is 45-50 nM; and/or, the reaction system further comprises an ssRNA fluorescent probe, the ssRNA fluorescent probe comprises, for example, a nucleotide sequence as shown in SEQ ID NO: 7; and/or, the working concentration of the ssRNA fluorescent probe is 0.25-0.5 µM.

10. A method for CRISPR detection, comprising a step of adding a sample to be tested to the reaction system of claim 9 to conduct a reaction;
preferably, the reaction temperature is 35-37°C; and/or, the CRISPR detection is detection for non-diagnostic purposes.

11. A method for detecting *Mycobacterium tuberculosis,* comprising steps of sequentially performing RNA nucleic acid-dependent amplification and Cas13a-based CRISPR detection on a sample to be tested; the RNA nucleic acid-dependent amplification comprises performing RNA amplification using the primer pair of claim 1 or 2, the nucleic acid detection kit of claim 3, or the reaction system of claim 4;
preferably, the CRISPR detection comprises performing detection using the crRNA of claim 6 or 7, the CRISPR detection kit of claim 8 or the reaction system of claim 9; and/or, the *Mycobacterium tuberculosis* is viable *Mycobacterium tuberculosis;* and/or, the method is for non-diagnostic purposes; and/or, the method is performed *in vitro;*
more preferably, the reaction temperature for the RNA amplification is 41-42°C; and/or, a thermal activation step is further comprised before the RNA amplification; the temperature of the thermal activation is preferably 65-67°C; and/or, the reaction temperature for the CRISPR detection is 35-37°C.

12. Use of the primer pair of claim 1 or 2, the nucleic acid detection kit of claim 3, the reaction system of claim 4, the crRNA of claim 6 or 7, the CRISPR detection kit of claim 8 or the reaction system of claim 9 in the detection of pathogens of *Mycobacterium* or in the manufacture of a reagent or device for detecting pathogens of *Mycobacterium;*
preferably, the pathogen of *Mycobacterium* is selected from *Mycobacterium tuberculosis, Mycobacterium gordonae, Mycobacterium abscessus, Mycobacterium fortuitum, Mycobacterium avium, Mycobacterium intracellulare and Mycobacterium kansasii.*
